# EUROPEAN PATENT SPECIFICATION

(11) **EP 1 567 669 B1**
(45) Date of publication and mention of the grant of the patent: **24.03.2010**
(21) Application number: 03782587.4
(22) Date of filing: 02.12.2003
(51) Int. Cl.: C12Q 1/68, B01J 19/00

(54) **DETERMINATION OF METHYLATION OF NUCLEIC ACID SEQUENCES**
BESTIMMUNG DER METHYLIERUNG VON NUKLEINSÄURESEQUENZEN
DETERMINATION DE LA METHYLATION DE SEQUENCES D'ACIDE NUCLEIQUE

(30) Priority: 02.12.2002 US 430315 P
(43) Date of publication of application: 31.08.2005
(73) Proprietor: Illumina Cambridge Limited, Little Chesterford Nr Saffron Walden Essex CB10 1XL (GB)
(72) Inventor: GORMLEY, Niall Solexa Ltd. Chesterford Res. Park, Essex CB10 1XL (GB)
(74) Representative: Barnes, Colin Lloyd
(86) International application number: PCT/GB2003/005263
(87) International publication number: WO 2004/050915

(56) References cited:
- EP-A- 1 180 548
- WO-A-01/94544
- US-A- 5 874 260
- PAUL C L ET AL: "Cytosine methylation: quantitation by automated genomic sequencing and GENESCAN analysis" BIOTECHNIQUES, EATON PUBLISHING, NATICK, US, vol. 21, no. 1, July 1996 (1996-07), pages 126-133, XP002143107 ISSN: 0736-6205
- FEIL R ET AL: "Methylation analysis on individual chromosomes: improved protocol for bisulphite genomic sequencing" NUCLEIC ACIDS RESEARCH, OXFORD UNIVERSITY PRESS, SURREY, GB, vol. 22, no. 4, 1994, pages 695-696, XP002106413 ISSN: 0305-1048

## Description

### BACKGROUND

In many eukaryotes, between 10 and 30% of cytosine bases are modified by the enzymatic addition of a methyl group. Although this modification does not interfere with the fidelity of DNA replication processes, it enables modulation of diverse cellular processes through protein interactions with hypo- or hyper-methylated sequences. These methylated sequences are not randomly dispersed throughout a genome, but instead, are almost exclusively found in repetitive CpG sequences in the regulatory regions upstream of many genes. Methylation of these sequences is associated with repression of gene activity and can result in global changes to gene expression. For example, methylation plays a central role in the inactivation of one of the two X chromosomes in female cells, which is a prerequisite for ensuring that females do not produce twice the level of X-linked gene products as would males. Methylation also underlies the selective repression of either the maternally or paternally inherited copy of pairs of alleles in a process known as genetic imprinting. It also silences transposable elements whose expression would otherwise be deleterious to a genome.

Patterns of methylation in a genome are heritable because of the semi-conservative nature of DNA replication. During this process, the daughter strand, newly replicated on a methylated template strand, is not initially methylated, but the template strand directs methyltransferase enzymes to fully methylate both strands. Thus, methylation patterns carry an extra level of genetic information down through the generations in addition to that information inherited in the primary sequence of the four nucleotides.

Aberrant patterns of genomic methylation also correlate with disease states and are among the earliest and most common alteration found in human malignancies. Moreover, mistakes made during the establishment of methylation patterns during development underlie several specific inherited disorders. Consequently, there is a demand for high throughput approaches for profiling the methylation status of many genes in parallel both for research purposes and for clinical applications.

Many methods already exist for detecting the methylation of DNA and they can be broadly classified depending on the level of sequence-specific information they produce. On the simplest level, there are techniques that only yield information on overall levels of methylation within a genome. For example, methylated sequences can be separated from unmethylated sequences on reverse-phase HPLC due to the difference in hydrophobicity of DNase I treated DNA. Such methods are simple but do not give any information on the sequence context of the methylation sites. Alternatively, pairs of restriction endonucleases that recognize the same sequence but have different sensitivities to cytosine methylation at that sequence can be used. Methylation at this sequence will render it refractory to cleavage by one enzyme, but sensitive to the other. If no cytosine bases are methylated in a sequence, both enzymes will produce identically sized restriction fragments. In contrast, if methylation is present, the enzymes will produce different sizes of fragments that can be distinguished by standard analytical techniques such as electrophoresis through agarose. If Southern blot analysis is subsequently performed and the bands probed with a labelled fragment from a gene of interest, then information on the sequence context of the methylation site can be investigated. These methods are limited because they are dependent on the availability of useful restriction enzymes and are confined to the study of methylation patterns among sequences that contain those restriction sites.

Methods of determining cytosine methylation are disclosed e.g. by Paul, C.L and Clark, S.J., Biotechniques 21: 126-133 (1996).

Methods that do not rely on sequence context but which can detect methylation at any chosen sequence are mainly based on the sodium bisulfite reaction. Under controlled conditions, this reagent converts cytosine to uracil while methyl-cytosine remains unmodified. If the treated DNA is then sequenced, the detection of a cytosine indicates that the cytosine is methylated because it would have been otherwise converted to a uracil.

Standard Sanger sequencing procedures have the disadvantage that only a limited number of sequencing reactions can be performed at the same time. Moreover, PCR amplification and sub-cloning may be necessary to produce sufficient quantities of DNA for sequencing, and both methods can introduce artifacts into the sequence, including changes in methylation.

Microarrays are molecular probes such as nucleic acid molecules arranged systematically onto a solid, generally flat surface. Each probe site carries a reagent such as a single stranded nucleic acid, whose molecular recognition of a complementary nucleic acid molecule leads to a detectable signal, often based on fluorescence. Microarrays carrying many thousands of probe sites can be used to monitor gene expression profiles over a large number of genes in a single experiment on a hybridisation based format.

The nucleic acid probes on the microarrays are generally made in two ways. A combination of photochemistry and DNA synthesis allows base-by-base synthesis of the probes *in situ*. This is the approach pioneered by Affymetrix for growing short strands of around 25 bases. Their 'genechips' are commercially available and widely used (*e*.*g*., Wodlicka et al., 1997, Nature Biotechnology 15:1359-1367), despite the expense of making arrays designed for a particular experiment. Another method for preparing microarrays is to use a robot to spot small (nL) volumes of nucleic acid sequences onto discreet areas of the surface. Microarrays prepared in this manner have less dense features than Affymetrix arrays but are more universal and cheaper to prepare (*e*.*g*., Schena et al., 1995, Science 270:467-470). The main drawback of all types of standard microarrays is the complex hardware required to achieve a spatial distribution of multiple copies of the same DNA sequence. Such limitations are overcome by single molecule array technology, *e*.*g*., as described in International Patent App. WO 00/06770.

In addition to hybridisation-based detection a number of other biochemical assays have been applied to nucleic acid microarrays, particularly in the area of genotyping. A common assay is to use a DNA polymerase or DNA ligase to incorporate a fluorescent marker onto the array. The enzyme incorporation allows the identity of one or more bases to be determined based on the identity of the labelled marker. Such extension assays have been developed by a number of companies and academic groups for typing single nucleotide polymorphisms ("SNPs"). The ability to perform multiple cycles of extension reactions on these platforms would be advantageous as it gives more information about the nature of the sample under investigation. For example, performing multiple extensions complementary to a template strand yields information on the sequence of the template strand. During such a 'sequencing by synthesis' reaction, a new strand, base-paired to the template nucleic acid, is built up in the 5' to 3' direction by incorporation of individual nucleotides complementary to those nucleotides in the template starting at its 3' end. The end result of a series of such incorporations is that the single-stranded template nucleic acid is no longer single-stranded; instead, it is base-paired to a synthetic complementary strand. The result is a double-stranded nucleic acid molecule: the original template nucleic acid and its complementary strand, attached to the solid substrate.

Once such a sequencing reaction is complete, removal of the synthetic strand complementary to the template would permit re-use of the template nucleic acid, *e*.*g*., in another sequencing reaction to verify the results of the first reaction. In another application, the sequenced strand becomes available for hybridization of nucleic acid, *e*.*g*., DNA or DNA mimics, *e*.*g*., PNA.

In contrast, the complete removal of both the template strand and its synthetic complement would allow new template nucleic acids to be attached to the solid substrate to form a new array.

### SUMMARY OF THE INVENTION

The invention relates to a method of detecting the precise locations of methyl-cytosines in a given nucleic acid sequence. In particular, the invention features a method which includes sequencing a template nucleic acid that is attached to a hairpin nucleic acid or double-stranded nucleic acid anchor. The template nucleic acid is then regenerated to single-stranded form via methods described herein, and then treated with sodium bisulfite, which converts the cytosines in the template nucleic acid to uracils unless the cytosines are methylated, in which case they remain as cytosines. The template nucleic acid is then resequenced. The results of the first and second sequencing reactions are then compared. The presence of a cytosine in the first sequence and a uracil in the corresponding location in the second sequence indicates that the cytosine at that location is unmethylated. However, the presence of a cytosine at a particular location in both the first second sequence indicates that the cytosine at that location is a methyl-cytosine.

The invention makes use of a hairpin nucleic acid, or a double-stranded nucleic acid anchor, which allows templates to be regenerated according to the invention. In particular, the hairpin nucleic acid or double-stranded nucleic acid anchor contains a restriction site, preferably for a nicking endonuclease, located before or at the 3' end of the hairpin nucleic acid. The hairpin nucleic acid or double-stranded nucleic acid anchor allow the regeneration of a single-stranded nucleic acid template following its conversion to a double-stranded product, *e*.*g*., as a result of a sequencing reaction.

The invention features a method for detecting a methylated cytosine in a template nucleic acid, the method including: (a) providing a hairpin-template complex, including: (i) a hairpin nucleic acid, where the hairpin nucleic acid is self-complementary and has a first restriction site for a nicking endonuclease, the restriction site including a recognition sequence and a cleavage site, where the recognition sequence is situated so that the cleavage site is before, at, or beyond the 3' end of the hairpin nucleic acid, and where the hairpin nucleic acid is a self-hybrid; and (ii) a single-stranded template nucleic acid; where 5' end of the hairpin nucleic acid is attached to the 3' end of the single-stranded template nucleic acid; (b) sequencing the single-stranded template nucleic acid of the hairpin-template complex, thereby producing: (ii) a first sequence; and (i) a hairpin-template-complement complex, including the hairpin-template complex of (a), and further including a synthetic nucleic acid strand complementary to the template nucleic acid, where the synthetic nucleic acid strand is hybridized to the template nucleic acid, and where the complementary nucleic acid strand is attached at its 5' end to the 3' end of the hairpin nucleic acid; (c) removing the complementary nucleic acid strand from the hairpin-template-complement complex, thereby recovering the hairpin-template complex, wherein the removal is carried out by a process comprising the use of the said nicking endonuclease which binds to said recognition site in said hairpin and cleaves the hairpin-template-complement complex at a site between the hairpin and the template complement; (d) treating the hairpin-template complex with sodium bisulfite, thereby producing a sodium bisulfite-treated template nucleic acid; (e) sequencing the sodium bisulfite-treated template nucleic acid of (c), thereby producing a second sequence; and (f) comparing the first sequence and the second sequence, where the presence of a cytosine in the second sequence indicates that the cytosine at that position is methylated; thereby detecting a methylated cytosine in the template nucleic acid. The hairpin nucleic acid can be attached to a solid substrate.

The disclosure also features an addressable single molecule array including a hairpin-template complex, including: (a) a hairpin nucleic acid, where the hairpin nucleic acid is self-complementary and has a first restriction site for a nicking endonuclease, the restriction site including a recognition sequence and a cleavage site, where the recognition sequence is situated so that the cleavage site is before, at, or beyond the 3' end of the hairpin nucleic acid, and where the hairpin nucleic acid is a self-hybrid, and where the hairpin nucleic acid is attached to a solid substrate; and (b) a single-stranded template nucleic acid, where the 5' end of the hairpin nucleic acid is attached to the 3' end of the single-stranded template nucleic acid. Such a single molecule addressable array can include a plurality of the hairpin-template complexes, where adjacent complexes are separated by a distance of at least 10nm, at least 100nm, or at least 250nm. The addressable array can include complexes at a density of 10⁶ to 10⁹ polynucleotides per cm², or 10⁷ to 10⁸ molecules per cm².

The disclosure also features a kit that includes such addressable arrays.

In a further aspect, the invention features a method for detecting a methylated cytosine in a template nucleic acid, the method including: (a) providing an anchor-template complex, including: (i) a double-stranded nucleic acid anchor, where the double-stranded nucleic acid anchor includes: (A) a first end and a second end; and (B) a first restriction site for a nicking endonuclease, the restriction site including a recognition sequence and a cleavage site, where the cleavage site is situated so that the cleavage site is before, at, or beyond the 3' end of the first end of the double-stranded nucleic acid anchor; and (ii) a single-stranded template nucleic acid; where the 5' end of the first end of the double-stranded nucleic acid anchor is attached to the 3' end of the single-stranded template nucleic acid; (b) sequencing the single-stranded template nucleic acid of the anchor-template complex, thereby producing: (i) a first sequence; and (ii) an anchor-template-complement complex, including the anchor-template complex of (a), and further including a synthetic nucleic acid strand complementary to the template nucleic acid, where the synthetic nucleic acid strand is hybridized to the template nucleic acid, and where the complementary nucleic acid strand is attached at its 5' end to the 3' end of the first end of the double-stranded nucleic acid anchor; (c) removing the complementary nucleic acid strand from the anchor-template-complement complex, thereby recovering the anchor-template complex, wherein the removal is carried out by a process comprising the use of the said nicking restriction endonuclease which binds to said recognition site in said anchor and cleaves the anchor-template-complement complex at a site between the anchor and the template complement; (d) treating the anchor-template complex with sodium bisulfite, thereby producing a sodium bisulfite-treated anchor-template complex; (e) sequencing the sodium bisulfite-treated anchor-template complex of (d), thereby producing a second sequence; and (f) comparing the first sequence and the second sequence, where the presence of a cytosine in the second sequence indicates that the cytosine at that position in the template nucleic acid is methylated; thereby detecting a methylated cytosine in the template nucleic acid. The double-stranded nucleic acid anchor can be attached at its second end to a solid substrate.

The disclosure additionally features an addressable single molecule array including an anchor-template complex, including: (a) a double-stranded nucleic acid anchor, where the double-stranded nucleic acid anchor includes: (i) a first end and a second end; and (ii) a first restriction site for a nicking endonuclease, the restriction site including a recognition sequence and a cleavage site, where the cleavage site is situated so that the cleavage site is before, at, or beyond the 3' end of the first end of the double-stranded nucleic acid anchor; and (b) a single-stranded template nucleic acid; where the 5' end of the first end of the double-stranded nucleic acid anchor is attached to the 3' end of the single-stranded template nucleic acid. Such an addressable single molecule array can include a plurality of the anchor-template complexes, where adjacent complexes are separated by a distance of at least 10nm, at least 100nm, or at least 250nm. The addressable array can contain complexes in a density of 10⁶ to 10⁹ polynucleotides per cm², or 10⁷ to 10⁸ molecules per cm².

The disclosure also features a kit including such an addressable array.

In another aspect, the disclosure features a method for detecting a methylated cytosine in a template nucleic acid of known sequence, the method including: (a) providing a hairpin-template complex, including: (i) a hairpin nucleic acid, where the hairpin nucleic acid is self-complementary and has a first restriction site for a nicking endonuclease, the restriction site including a recognition sequence and a cleavage site, where the recognition sequence is situated so that the cleavage site is before, at, or beyond the 3' end of the hairpin nucleic acid, and where the hairpin nucleic acid is a self-hybrid; and (ii) a single-stranded template nucleic acid; where 5' end of the hairpin nucleic acid is attached to the 3' end of the single-stranded template nucleic acid; (b) treating the hairpin-template complex with sodium bisulfite, thereby producing a sodium bisulfite-treated template nucleic acid; (c) sequencing the sodium bisulfite-treated template nucleic acid of (b), thereby producing a sequence; and (d) comparing the sequence of (c) and the known sequence, where the presence of a cytosine in the sequence of (c) indicates that the cytosine at that position is methylated; thereby detecting a methylated cytosine in the template nucleic acid of known sequence. The hairpin nucleic acid can be attached to a solid substrate.

The disclosure further features a method for detecting a methylated cytosine in a template nucleic acid of known sequence, the method including: (a) providing an anchor-template complex, including: (i) a double-stranded nucleic acid anchor, where the double-stranded nucleic acid anchor includes: (A) a first end and a second end; and (B) a first restriction site for a nicking endonuclease, the restriction site including a recognition sequence and a cleavage site, where the cleavage site is situated so that the cleavage site is before, at, or beyond the 3' end of the first end of the double-stranded nucleic acid anchor; and (ii) a single-stranded template nucleic acid; where the 5' end of the first end of the double-stranded nucleic acid anchor is attached to the 3' end of the single-stranded template nucleic acid; (b) treating the anchor-template complex with sodium bisulfite, thereby producing a sodium bisulfite-treated anchor-template complex; (c) sequencing the sodium bisulfite-treated anchor-template complex of (b), thereby producing a sequence; and (d) comparing the sequence of (c) and the known sequence, where the presence of a cytosine in the sequence of (c) indicates that the cytosine at that position in the template nucleic acid is methylated; thereby detecting a methylated cytosine in the template nucleic acid. The double-stranded nucleic acid anchor can be attached at its second end to a solid substrate.

The disclosure also features a method for detecting a methylated cytosine in a template nucleic acid of known sequence, where one or more of the cytosines in the template nucleic acid have been converted to uracil, the method including: (a) providing a hairpin-template complex, including: (i) a hairpin nucleic acid, where the hairpin nucleic acid is self-complementary and has a first restriction site for a nicking endonuclease, the restriction site including a recognition sequence and a cleavage site, where the recognition sequence is situated so that the cleavage site is before, at, or beyond the 3' end of the hairpin nucleic acid, and where the hairpin nucleic acid is a self-hybrid; and (ii) a single-stranded template nucleic acid; where 5' end of the hairpin nucleic acid is attached to the 3' end of the single-stranded template nucleic acid; (b) sequencing the template nucleic acid, thereby producing a sequence; and (c) comparing the sequence of (b) and the known sequence, where the presence of a cytosine in the sequence of (b) indicates that the cytosine at that position is methylated; thereby detecting a methylated cytosine in the template nucleic acid of known sequence. The hairpin nucleic acid can be attached to a solid substrate.

The disclosure features in an additional aspect a method for detecting a methylated cytosine in a template nucleic acid of known sequence, where one or more of the cytosines in the template nucleic acid have been converted to uracil, the method including: (a) providing an anchor-template complex, including: (i) a double-stranded nucleic acid anchor, where the double-stranded nucleic acid anchor includes: (A) a first end and a second end; and (B) a first restriction site for a nicking endonuclease, the restriction site including a recognition sequence and a cleavage site, where the cleavage site is situated so that the cleavage site is before, at, or beyond the 3' end of the first end of the double-stranded nucleic acid anchor; and (ii) a single-stranded template nucleic acid; where the 5' end of the first end of the double-stranded nucleic acid anchor is attached to the 3' end of the single-stranded template nucleic acid; (b) sequencing the anchor-template complex, thereby producing a sequence; and (c) comparing the sequence of (b) and the known sequence, where the presence of a cytosine in the sequence of (b) indicates that the cytosine at that position in the template nucleic acid is methylated; thereby detecting a methylated cytosine in the template nucleic acid. The double-stranded nucleic acid anchor can be attached at its second end to a solid substrate.

The disclosure features a hairpin nucleic acid, having the following characteristics: (a) being self-complementary; and (b) having a first restriction site for a nicking endonuclease, the restriction site including a recognition sequence and a cleavage site, where the recognition sequence is situated so that the cleavage site is before, at, or beyond the 3' end of the hairpin nucleic acid. The hairpin nucleic acid can further include one or more modifications to allow hairpin nucleic acid attachment to a solid substrate. The hairpin nucleic acid can also further include a second restriction site for a blunt-end endonuclease, the second restriction site including a second recognition sequence and a second cleavage site, where the second recognition sequence is situated so that the second cleavage site is before, at, or beyond the 3' end of the hairpin nucleic acid.

The disclosure also features a method for recovering a single-stranded template nucleic acid, the method including: (a) providing a single-stranded template nucleic acid attached to the 5' end of a hairpin nucleic acid, where the hairpin nucleic acid is self-complementary and has a first restriction site for a nicking endonuclease, the restriction site including a recognition sequence and a cleavage site, where the recognition sequence is situated so that the cleavage site is before, at, or beyond the 3' end of the hairpin nucleic acid, and where the hairpin nucleic acid is a self-hybrid, and where a nucleic acid strand complementary to the template nucleic acid is attached to the 3' end of the hairpin nucleic acid; (b) contacting the hairpin nucleic acid with the nicking endonuclease, under conditions where the nicking endonuclease cleaves before, at or beyond the 3' end of the hairpin nucleic acid, thereby providing a nicked hairpin-template-complement nucleic acid complex; and (c) subjecting the nicked hairpin-template-complement nucleic acid complex to conditions whereby the nucleic acid strand complementary to the template nucleic acid dissociates from the template nucleic acid; thereby recovering the single-stranded template nucleic acid. The hairpin nucleic acid can be attached to a solid substrate.

In another aspect, the disclosure features an addressable single molecule array, including a hairpin nucleic acid as described above, where the hairpin nucleic acid is attached to a solid substrate. Adjacent hairpin nucleic acids in such an array can be separated by a distance of at least 10nm, of at least 100nm, or of at least 250nm. The density of the hairpin nucleic acids can be from 10⁶ to 10⁹ polynucleotides per cm², or from 10⁷ to 10⁸ molecules per cm².

The disclosure also features a kit including a hairpin nucleic acid as described above, and packaging components therefor. The disclosure also features a kit which includes an addressable array as described above.

In another aspect, the disclosure features a double-stranded nucleic acid anchor, having the following characteristics: (a) having a first end and a second end; and (b) having a first restriction site for a nicking endonuclease, the restriction site including a recognition sequence and a cleavage site, where the recognition sequence is situated so that the cleavage site is located before, at, or beyond the 3' end of the first end of the double-stranded nucleic acid anchor. The double-stranded nucleic acid anchor can be attached at its second end to a solid substrate. The double-stranded nucleic acid anchor can further include a second restriction site for a blunt-end endonuclease, the second restriction site including a second recognition sequence and a second cleavage site, where the second recognition sequence is situated so that the second cleavage site is located before, at, or beyond the 3' end of the first end of the double-stranded nucleic acid anchor.

The disclosure also features a method for recovering a single-stranded template nucleic acid, the method including: (a) providing a single-stranded template nucleic acid attached to a double-stranded nucleic acid anchor, and where a nucleic acid strand complementary to the template nucleic acid is attached to the double-stranded nucleic acid anchor, and where the double-stranded nucleic acid anchor: (i) has a first end and a second end; and (ii) has a first restriction site for a nicking endonuclease, the restriction site including a recognition sequence and a cleavage site, where the cleavage site is situated so that the cleavage site is before, at, or beyond the 3' end of the first end of the double-stranded nucleic acid anchor; where the single-stranded template nucleic acid is attached to the 5' end of the first end of the double-stranded nucleic acid anchor, and where the nucleic acid strand complementary to the template nucleic acid is attached to the 3' end of the first end of the double-stranded nucleic acid anchor; (b) contacting the double-stranded nucleic acid anchor with the nicking endonuclease, under conditions where the nicking endonuclease cleaves before, at, or beyond the 3' end of the first end of the double-stranded nucleic acid anchor, thereby providing a nicked anchor-template-complement nucleic acid complex; and (c) subjecting the nicked anchor-template-complement nucleic acid complex to conditions whereby the nucleic acid strand complementary to the template nucleic acid dissociates from the template nucleic acid; thereby recovering the single-stranded template nucleic acid. The double-stranded nucleic acid anchor can be attached at its second end to a solid substrate.

In another aspect, the disclosure features an addressable single molecule array, including a double-stranded nucleic acid anchor as described above, where the double-stranded nucleic acid anchor is attached to a solid substrate. Adjacent double-stranded nucleic acid anchors in such an array can be separated by a distance of at least 10nm, of at least 100nm, or of at least 250nm. The density of the double-stranded nucleic acid anchors can be from 10⁶ to 10⁹ polynucleotides per cm², or from 10⁷ to 10⁸ molecules per cm².

The disclosure also features a kit including a double-stranded nucleic acid anchor as described above, and packaging components therefor. The disclosure also features a kit which includes an addressable array as described above.

By "methylated cytosine" is meant a cytosine with an added methyl group on the carbon 5 position.

"First sequence" and "second sequence", as used herein, refer to the information regarding the sequential nucleotides in a nucleic acid sequence, presented in text, computer-readable, or other non-biological form, that is, the terms refer to the sequence information, rather than to the physical nucleic acids themselves. By "first" and "second" sequences is meant the results of a first sequencing reaction and a second sequencing reaction. The results of the two sequencing reactions (the first and second sequences, respectively), are then compared.

By "comparing the first sequence and the second sequence" is meant that the sequential nucleotide information resulting from the first sequencing reaction is compared to the sequential nucleotide information resulting from the second sequencing reaction, and the differences between the two are noted. In the case where the template strand is sequenced, and then treated with sodium bisulfite (thereby converting the unmethylated cytosines to uracils), the presence of a cytosine at a particular location in the first sequence and a cytosine in the same location in the second sequence indicates that that particular cytosine is methylated in the original template nucleic acid. The presence of a cytosine at a particular location in the first sequence and the presence of a uracil in the same location in the second sequence indicates that that particular cytosine is a unmethylated in the original template nucleic acid.

"By "treating the hairpin-template-complex with sodium bisulfite" is meant that the hairpin-template-complex is contacted with an amount of sodium bisulfite under conditions whereby the unmethylated cytosines in the template nucleic acid will be chemically modified and converted to uracils. The actual protocol for treating the template nucleic acid with sodium bisulfite can be any of those known in the art, or as provided herein.

Alternatively, other methods of differentiating between the two can be used, *e*.*g*., a chemical (or other) treatment that reliably converts either the cytosines or the methylated cytosines to another, specific nucleotide can be used, and the differences between the results of the two sequencing reactions can be compared. For instance, a method of chemical modification can be used which converts cytosine to a different nucleotide, and the differences in the results of two sequencing reactions can be compared. Alternatively, a method of chemical modification can be used which converts methyl-cytosine to a different nucleotide, and the differences in the results of two sequencing reactions can be compared.

The method can also be used to detect the presence of other modified nucleotides in a nucleic acid, given a method (chemical or otherwise, *e*.*g*., enzymatic, etc.) of specifically treating the modified nucleotides so that a subsequent sequencing reaction produces a sequence that is changed relative to the first sequencing reaction.

In one embodiment, "hairpin nucleic acid" means a single-stranded nucleic acid which is capable of forming a hairpin, that is, a nucleic acid whose sequence contains a region of internal self-complementarity enabling the formation of an intramolecular duplex or self-hybrid. "Region of self-complementarity" refers to self-complementarity over a region of 4 to 100 base pairs. When not self-hybridized, the hairpin nucleic acid can be 8 to 200 base pairs, preferably 10 to 30 base pairs in length. By saying that the hairpin nucleic acid is a "self-hybrid", or that the hairpin nucleic acid has "self-hybridized", means that the hairpin nucleic acid has been exposed to conditions that allow its regions of self-complementarity to hybridize to each other, forming a double-stranded nucleic acid with a loop structure at one end and an exposed 3' and 5' end at the other. It is preferable, but not required, that when hybridized to itself, the exposed 3' and 5' ends form a blunt end.

The hairpin nucleic acid can also possess one or more moieties which allow the hairpin nucleic acid to be attached to a solid substrate. Generally, such moieties will be located together in the vicinity of the center of the hairpin nucleic acid, so that when the hairpin nucleic acid has self-annealed, the moiety is located at the bend in the hairpin, allowing the bend to be attached to a solid substrate. The hairpin can be self-hybridized before or after attachment to the substrate.

In one embodiment, the hairpin nucleic acid is a molecular stem and loop structure formed from the hybridisation of complementary polynucleotides. The stem comprises the hybridized polynucleotides and the loop is the region that covalently links the two complementary polynucleotides. Anything from a 4 to 100 base pair double-stranded (duplex) region may be used to form the stem.

In another embodiment, the hairpin nucleic acid is a molecule which is synthesized in a contiguous fashion but is not made up entirely of DNA, rather the ends of the molecule comprise DNA bases that are self-complementary and can thus form an intramolecular duplex, while the middle of the molecule includes one or more non-nucleic acid molecules. An example of such a hairpin nucleic acid would be Nu-Nu-Nu-Nu-Nu-LM-Nc-Nc-Nc-Nc-Nc, where "Nu" is a particular nucleotide, "Nc" is the nucleotide complementary to Nu, and "LM" is the linker moiety linking the two strands, *e*.*g*., hexaethylene glycol (HEG) or polyethylene glycol (PEG). The non-nucleic acid molecule(s) can be linker moieties for linking the two nucleic acids together (the two nucleic acid halves of the overall hairpin nucleic acid), and can also be used to attach the overall hairpin nucleic acid to the substrate. Alternatively, the non-nucleic acid molecule(s) can be intermediate molecules which are in turn attached to linker moieties used for attaching the overall hairpin nucleic acid to the solid substrate.

In another embodiment, the hairpin nucleic acid is composed of two separate but complementary nucleic acid strands that are hybridized together to form an intermolecular duplex, and are then covalently linked together. The linkage can be accomplished by chemical crosslinking of the two strands, attaching both strands to one or more intercalators or chemical crosslinkers, etc.

By "double-stranded nucleic acid anchor", or "anchor", is meant a segment of double-stranded nucleic acid which, like the hairpin nucleic acid described above, is designed to contain one or more restriction sites capable of being acted on by one or more restriction endonucleases, *e*.*g*., a nicking endonuclease. The double-stranded nucleic acid anchor will have a first end and a second end. The first end is used for attachment of the template nucleic acid and the strand complementary to the template nucleic acid. The second end of the double-stranded nucleic acid anchor can possess one or more nucleotides which are modified to allow the double-stranded nucleic acid anchor to be attached to a solid substrate. Because the anchor is double-stranded, both the first end and the second end will each have a strand with a 3' end, and a strand with a 5' end. The anchor can be a double-stranded oligonucleotide bonded to the substrate, or two single-stranded oligonucleotides bonded to the substrate and than hybridized.

Thus, the terms "hairpin," "hairpin nucleic acid," and "double-stranded nucleic acid anchor" include cross-linked (*e*.*g*., hybridized, chemically cross-linked, etc.) duplex nucleic acids or nucleic acid mimics (*e*.*g*., peptide nucleic acids (PNA)) which are capable of being recognized and acted upon by endonucleotides and polymerses.

The hairpin nucleic acids and double-stranded nucleic acid anchors generally exist as molecules in solution before being attached to the solid substrate. In the case of hairpin nucleic acids, the hairpin nucleic acid can be hybridized to itself before or after it is attached to the substrate. In the case of double-stranded nucleic acid anchors, the two nucleic acid strands of the anchor can be hybridized together, and the anchor then attached to the substrate, or the individual single stranded components of the anchor can be attached to the surface, and then hybridized together.

The hairpin nucleic acids and double-stranded nucleic acid anchors (whether self-byridized or not) can be attached to the substrate in any way known in the art. Generally, such methods involve modifying the nucleic acid such that it contains a chemical group or biochemical or other molecule (*e*.*g*., biotin or streptavidin, etc.) that is either inherently reactive with the substrate or can be activated to bond to the substrate. Modifications can be made to any part of the nucleic acid, including linkers being attached to the bases, sugars, phosphates, or at the 3' and 5' hydroxyl groups. Modification can be made at any part of the hairpin nucleic acid or double-stranded nucleic acid anchor to achieve surface attachment.

By saying that an endonuclease cuts "before, at or beyond the 3' end" of a hairpin nucleic acid, means that the "restriction site" for a given endonuclease comprises both a "recognition sequence" and a "cleavage site". The recognition sequence is the precise sequence of nucleotides recognized by a particular endonuclease, *e*.*g*., the recognition sequence for nicking endonuclease N.BbvCIA is "GCTGAGG" (see Table 1). The cleavage site for this endonuclease is within this recognition sequence, between the "C" and the "T". The recognition sequence for N.BstNBI is "GAGTCNNNN", where "N" can be any nucleotide. The precise recognition sequence is therefore effectively "GAGTC". The cleavage site for this endonuclease is four nucleotides 3' from the end of this recognition sequence.

There is no requirement that the restriction site be situated so that the endonuclease cuts or nicks exactly at the 3' end of the hairpin nucleic acid. The cleavage site can lie within the hairpin nucleic acid, lie at the very end of the hairpin nucleic acid, or lie outside of it.

There exist nicking endonucleases that nick (cleave) at a position 3' of the recognition sequence, that is, the recognition sequence and the cleavage site are separated by several (*e*.*g*., 4-5) nucleotides. Such nicking endonucleases include N.AlwI, N.BspD6I, N.Bst9I, N.BstNBI, N.BstSEI, where four random nucleotides separate the recognition sequence and the cleavage site, and N.MlyI, where five random nucleotides separate the recognition sequence and the cleavage site.

There is also no requirement that the recognition sequence be separated from the cleavage site. As shown in Table 1, there exist nicking endonucleases that cut (cleave) within their recognition sequence (*e*.*g*., N.BbvCIA, N.BbvCIB, N.Bpu10IA, N.Bpu10IB, N.CviPII, N.CviQXI), similar to the action of an ordinary restriction endonuclease (*i*.*e*., an enzyme that cleaves through both strands of a double stranded nucleic acid).

By saying that an endonuclease cuts "before" the 3' end of a hairpin nucleic acid means that the cleavage site for a particular endonuclease occurs before the 3' end of the hairpin nucleic acid, and that nucleotides will be removed from the 3' end of the hairpin nucleic acid. For instance, in the case of endonuclease N.BbvCIA, the placement of the recognition sequence for this endonuclease within a hairpin nucleic acid means that this endonuclease will, by definition, cleave at a point before the 3' end of the hairpin nucleic acid.

By saying that an endonuclease cuts "at" the 3' end of a hairpin nucleic acid means that the cleavage site is situated so that the endonuclease cleaves at a point exactly between the 3' end of the hairpin nucleic acid and any nucleotides or nucleic acid strand added to it. For instance, in the case of N.BstNBI, the restriction site is "GAGTCNNNN^". A hairpin nucleic acid that ends in the sequence ...GAGTCATGC-3' will be cut exactly at its 3' end by N.BstNBI, thereby removing any nucleotides incorporated onto the end of the hairpin.

By saying that an endonuclease cuts "beyond" the 3' end of a hairpin nucleic acid means that the cleavage site of the endonuclease cleaves at a point beyond the 3' end of the hairpin, between nucleotides that have been added to the hairpin. For instance, if a hairpin nucleic acid ends in the sequence ...GAGTC-3', and has a strand attached to it that begins with 5'-AATTGGCC..., then the endonuclease N.BstNBI will cut between T and G of the attached strand, that is, at GAGTC AATT^GGCC.

If the recognition sequence in the hairpin nucleic acid is that of a nicking endonuclease that cleaves within its recognition sequence, the inclusion of such a recognition sequence in a hairpin nucleic acid will result in the removal of several nucleotides (*i*.*e*., two in the case of N.CviPII, N.CviQXI; five in the case of N.BbvCIA, N.BbvCIB, N.Bpu10IA, N.Bpu10IB) from the 3' end of the hairpin. Depending on the intended use of the hairpin nucleic acid, such a loss may be acceptable, as after removal of the complementary strand, the limited number of nucleotides removed from the hairpin nucleic acid can be added back by using the same reaction as that used to build up the complementary strand in the first place.

Some enzymes may not be useful for all applications. For instance, N.CviPII and N.CviQXI have very short recognition sequences(C^CD and R^AG, respectively), which nick frequently, and may therefore nick the template itself. If the template is short, and does not contain these sequences, then these enzymes may be useful.

There is no requirement that the restriction site be situated so that the endonuclease cuts or nicks exactly at the 3' end of the first end of the double-stranded nucleic acid anchor. The endonuclease can cut or nick just before the 3' end, if it is not necessary that perfect integrity of the double-stranded nucleic acid anchor be maintained. The endonuclease can also cut or nick beyond the 3' end of the double-stranded nucleic acid anchor, if it is not detrimental that nucleotides be effectively added to the anchor.

If the recognition sequence in the hairpin nucleic acid is that of a nicking endonuclease that cleaves beyond the recognition sequence, the inclusion of such a recognition sequence in a hairpin nucleic acid will result in nicking of the strand at a location a few nucleotides beyond the recognition sequence. If the recognition sequence is located at the 3' end of the hairpin nucleic acid, then cleavage will occur 4-5 nucleotides beyond the end of the hairpin nucleic acid. If, however, the 3' end of the recognition sequence for any of N.AlwI, N.BspD6I, N.Bst9I, N.BstNBI and N.BstSEI is located four nucleotides from the end of the hairpin nucleic acid, then these enzymes will cut exactly at the end of the hairpin nucleic acid. If, however, the 3' end of the recognition sequence for any of these enzymes is located more than four nucleotides from the 3' end of the hairpin nucleic acid, then the nicking endonuclease will nick before the 3' end of the hairpin.

The endonuclease can cut or nick just before the 3' end of the hairpin, if it is not necessary that perfect integrity of the hairpin be maintained. The endonuclease can also cut or nick beyond the 3' end of the hairpin nucleic acid, if it is not detrimental that nucleotides be effectively added to the hairpin.

According to the invention, a hairpin nucleic acid is designed so that the restriction site for a nicking endonuclease is located so that the endonuclease will nick at a location before, at, or beyond the 3' end of the hairpin. The hairpin is then self-annealed and a single-stranded template nucleic acid is attached to the 5' end of the hairpin. After a sequencing or other reaction builds a synthetic strand complementary to the template nucleic acid, the synthetic complementary strand can be removed by (1) nicking with the nicking endonuclease that recognizes the restriction site within the hairpin, so that a nick is made at a point before, at or beyond the 3' end of the hairpin, effectively "disconnecting" the synthetic complementary strand from the hairpin, so that the two are no longer contiguous, and (2) washing away the synthetic complementary strand, by standard denaturation, *e*.*g*., heat, formamide, NaOH, etc..

Practice of the method of the invention with a double-stranded nucleic acid anchor is very similar to using a hairpin nucleic acid. The present application largely discusses use of hairpin nucleic acids in the invention, however, one of ordinary skill will readily understand that the double-stranded nucleic acid anchors can perform all of the same functions, and possess the same advantages over previous methods, as the hairpin nucleic acids.

It is to be understood that in stating that the cut made by the endonuclease is "before, at, or beyond" the 3' end of the hairpin, it is meant that the cut is made in the vicinity of the 3' end of the hairpin, and that the recognition sequence for the endonuclease is not located at the 5' end of the hairpin nucleic acid resulting in cleavage within the 5' half of the hairpin nucleic acid. It is also understood that by saying that the cut may be made "beyond" the 3' end of the hairpin nucleic acid, the distance beyond the 3' end is constrained by the distance between the recognition sequence and cleavage site for the given endonuclease. For instance, of the nicking endonucleases in Table 1, none nicks at a point farther than five nucleotides from the recognition sequence. Therefore, no cleavage will occur farther than five nucleotides beyond the end of the 3' end of the hairpin nucleic acid, unless endonucleases are used which have cleavage sites that are further removed from their recognition sequences.

The hairpin nucleic acid or the double-stranded nucleic acid anchor can be attached to a substrate, *e*.*g*., in a spatially-addressable array.

"Template nucleic acid," or "single-stranded template nucleic acid," as used herein, means a linear single-stranded nucleic acid molecule which, when attached to the self-annealed hairpin nucleic acid (or anchor) described herein, is capable of being recognized and acted upon by a polymerase such that, under the proper conditions, the polymerase incorporates nucleotides onto the 3' end of the hairpin nucleic acid, where each nucleotide is complementary to the corresponding nucleotide on the template nucleic acid, thereby extending the 3' end of the hairpin and producing a nucleic acid strand complementary to the template nucleic acid. The term also includes a double-stranded nucleic acid that is attached to the hairpin, where one strand is then removed, leaving a single strand. The term can also include the ligation and covalent attachment of both strands of a double-stranded nucleic acid to the hairpin nucleic acid or double-stranded nucleic acid anchor, followed by nicking according to the methods described herein followed by washing to remove the nicked strand, that is, the method of the invention can itself be used in the attachment of the template nucleic acid to the hairpin nucleic acid or the double-stranded nucleic acid anchor. Alternatively, one strand of a double-stranded nucleic acid can be ligated to the hairpin nucleic acid or double-stranded nucleic acid anchor, and the second strand washed away.

The template can be any length that can be successfully sequenced, preferably 10 to 100 nucleotides, more preferably 15 to 100 nucleotides, most preferably 20 to 30 nucleotides. Although the term "template nucleic acid" is used herein, it will be appreciated by one of ordinary skill that the invention is not limited to sequencing reactions, but that the techniques can be used to assay the interaction of the "templates" with other molecules. Such embodiments are described below.

By stating that the template is "attached" to the hairpin or anchor is meant that the template nucleic acid is covalently attached.

By stating that the polymerase will act upon the template and incorporate nucleotides onto the 3' end of the hairpin is meant that the polymerase will act given appropriate conditions, such as appropriate temperature, buffers, pH, nucleotides, and other reaction components and conditions required for action by the polymerase.

By "nucleic acid strand complementary to the template nucleic acid", or "synthetic nucleic acid strand complementary to the template nucleic acid", or more simply, "complement", is meant a strand of nucleic acid which possesses a sequence that is complementary to that of the template nucleic acid, that is, the complement and the template nucleic acids can hybridize and form a stretch of double-stranded nucleic acid.

By stating that the template or complement is "attached" to the hairpin or anchor is meant that the template nucleic acid or its complement are covalently attached.

As used herein, the term "array" refers to a population of hairpin nucleic acids or double-stranded nucleic acid anchors that are distributed over a solid support. The nucleic acids can be distributed in a single molecule array, that is the nucleic acids are spaced at a distance from one another sufficient to permit their individual resolution. Alternatively, nucleic acids of one type can be clustered at a single address, when one or more nucleic acids at the address can be detected.

"Solid support", as used herein, refers to the material to which the hairpins and/or anchors are attached. Suitable solid supports are available commercially, and will be apparent to the skilled person. The supports can be manufactured from materials such as glass, ceramics, silica and silicon. Supports with a gold surface may also be used. The supports usually comprise a flat (planar) surface, or at least a structure in which the molecules to be interrogated are in approximately the same plane. Alternatively, the solid support can be nonplanar, *e*.*g*., a microbead. Any suitable size may be used. For example, the supports might be on the order of 1-10 cm in each direction.

In one aspect of the invention, the "array" is a device comprising a "single molecule array," that is, a plurality of the hairpins and/or anchors of the invention, *i*.*e*., the hairpin and/or anchor molecules, are immobilized on the surface of a solid support, such that the molecules are at a density that permits individual resolution of at least two of the molecules and their attached templates. "Plurality" is used to mean that multiple molecules are placed on the array. The molecules can be of all the same type, or of multiple, *i*.*e*., different, types, *i*.*e*., the array can be composed entirely of hairpins, or entirely of anchors, or of a mixture of the two. In general, the hairpins/anchors are at a density of 10⁶ to 10⁹ individually resolvable polynucleotides per cm², preferably 10⁷ to 10⁹ individually resolvable polynucleotides per cm².

In another aspect of the invention, the "array" is a device comprising a high-density array, that is, where each individual address on the array comprises a cluster of nucleotides of the same type, while another address on the array comprises a cluster of nucleotides of a different type. Detection of an address is done by detecting one or more individual nucleotides at the address.

As used herein, the term "interrogate" means contacting one or more of the hairpins and/or anchors with another molecule, *e*.*g*., a polymerase, a nucleoside triphosphate, a complementary nucleic acid sequence, wherein the physical interaction provides information regarding a characteristic of the arrayed molecule and the template nucleic acid attached to it. The contacting can involve covalent or non-covalent interactions with the other molecule. As used herein, "information regarding a characteristic" means information regarding the sequence of one or more nucleotides in the template, the length of the template, the base composition of the template, the Tₘ of the polynucleotide, the presence of a specific binding site for a polypeptide or other molecule, the presence of an adduct or modified nucleotide, or the three-dimensional structure of the template.

The term "individually resolved by optical microscopy" is used herein to indicate that, when visualized, it is possible to distinguish at least one polynucleotide on the array from its neighbouring polynucleotides using optical microscopy methods available in the art. Visualisation may be effected by the use of reporter labels, *e*.*g*., fluorophores, the signal of which is individually resolved.

### BRIEF DESCRIPTION OF THE DRAWINGS

Fig. 1 is a diagram illustrating a hairpin-template-complement complex, and the recovery and regeneration of the template nucleic acid.
Fig. 2 is a diagram illustrating the steps in sequencing a single stranded nucleic acid template attached by a hairpin (or other anchoring sequence) to a substrate.
Fig. 3 is a diagram showing a hairpin containing a nicking site of the nicking endonuclease N.*Bst*NBI.
Fig. 4 is a diagram showing a hairpin containing a cleavage site of blunt end endonuclease *Mly*I.
Fig. 5 is a diagram showing a double-stranded nucleic acid anchor containing a nicking site of the nicking endonuclease N.*Bst*NBI.

### DETAILED DESCRIPTION

The present invention discloses a method of determining the presence and locations of methylated cytosines in a template nucleic acid sequence. The method comprises the steps of sequencing a template nucleic acid, treating it with sodium bisulfite to convert unmethylated cytosines to uracils, and then resequencing the template nucleic acid to determine at which positions methylated cytosines are present, that is, where cytosines are not converted to uracils. The method uses a method for regenerating a single-stranded nucleic acid template following its conversion to a double-stranded product, *e*.*g*., during a sequencing reaction. The invention also uses a method of removing a double-stranded nucleic acid from its substrate, *e*.*g*., removing a double stranded nucleic acid from another molecule anchoring it to a solid substrate, or from a hairpin nucleic acid anchoring the double stranded nucleic acid to a solid substrate.

Single-molecule sequencing allows complete genomes to be sequenced on a single microarray chip in a single sequencing reaction. The principle of this technology is that large numbers of short sequences from fragmented DNA are immobilized as single strands on a surface where they can be individually visualized with a sensitive microscope and camera. Every fragment is then sequenced simultaneously with fluorescent nucleotides and a polymerase enzyme, and the sequence information from all of the molecules is recorded simultaneously within a single camera frame. The method does not rely on DNA amplification by PCR or any sub-cloning steps, instead, tiny quantities of DNA can be directly sequenced immediately after being extracted from source. When a sequencing reaction is complete, the single stranded template strand can be regenerated by enzymatic cleavage of the newly synthesized sequencing strand as described herein. The DNA is then treated with sodium bisulfite that converts unmethylated cytosines to uracils. If a second sequencing reaction is then performed on the template, then the detection of cytosines will indicate that those bases are methylated.

Unlike many other methylation detection techniques, the sodium bisulfite method does not rely on the presence of a restriction site nor any prior knowledge of the sequence context. Furthermore, as provided herein, the single-stranded nature of the template DNA avoids potential artifacts arising from the sodium bisulfite reaction, which are found in prior art techniques. Sodium bisulfite will only react with pyrimidines that are not base-paired. Various technical modifications to sodium bisulfite reactions have been attempted by others to reduce strand annealing, but less than complete conversion of unmethylated cytosines to uracils can still occur resulting in incorrect interpretation of data.

As an alternative to such techniques, a pool of fragmented DNA can be split into two portions and immobilized as single strands on separate microarrays. One array can be treated with bisulfite and then both arrays sequenced. A comparison of the sequence data from the two arrays will indicate sites of methylation. This approach avoids the need to regenerate a sequencing template and requires only one sequencing reaction per microarray, although it requires the use of two microarrays and twice the amount of DNA.

Another alternative is to attach the template nucleic acids to hairpin nucleic acids or double-stranded nucleic acid anchors as described herein, which permit the recovery and regeneration of the original single-stranded template nucleic acid after it has been sequenced and converted to a double-stranded product. After such regeneration and recovery, the template nucleic acid can be treated with sodium bisulfite and resequenced, producing the second set of results on the same template nucleic acids on the same array.

The use of the methods described herein on a single-molecule array thus represents a technically simple procedure to assess methylation patterns across an entire genome without prior knowledge of restriction sites and without the artifacts of conventional bisulfite methodologies.

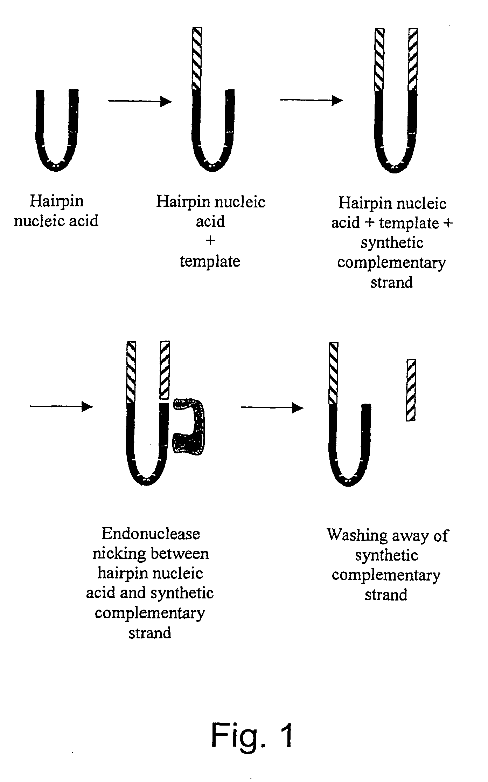

To regenerate the template nucleic acid between the two sequencing reactions, a hairpin nucleic acid containing a restriction site is provided, *i*.*e*., a single-stranded nucleic acid with a region of internal complementarity (*i*.*e*., is capable of hybridizing to itself and forming a hairpin) and also containing a restriction site. The hairpin nucleic acid has, near its 3' end, a restriction site for a nicking endonuclease. The restriction site is situated so that the nicking endonuclease will nick at a point before, at, or beyond the 3' end of the single-stranded nucleic acid. A nicking endonuclease acting upon such a restriction site in such a nucleic acid is shown in Fig. 1.

To use the hairpin to recover a template nucleic acid, a single-stranded nucleic acid template is attached to the 5' end of the hairpin. This can be done in a number of ways. A single-stranded nucleic acid can be attached to the hairpin. Alternatively, a double-stranded nucleic acid can be attached to the hairpin. Alternatively, a double-stranded nucleic acid can be attached to the hairpin, and either one strand ligated to the hairpin, or both strands can be ligated and then one strand removed, *e*.*g*., according to the methods described herein. The hairpin nucleic acid is then self-annealed to form a hairpin with an attached template nucleic acid. Alternatively, the hairpin can be self-annealed first, with the single-stranded template nucleic acid being then being attached to the hairpin. Once the template nucleic acid is attached to the hairpin, it is in a position to be "recovered" following a sequencing or other reaction that builds up a strand complementary to the template nucleic acid, and attached to the 3' end of the hairpin.

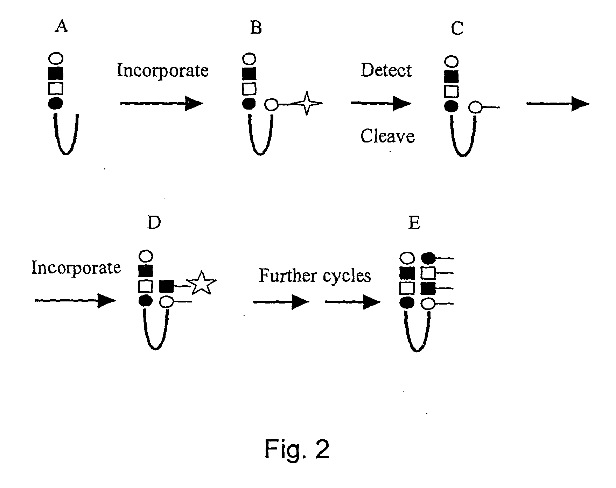

During such a reaction, such as that shown in Fig. 2, single nucleotides are generally incorporated onto the 3' end of the hairpin, where each nucleotide is complementary to the nucleotide opposite it on the template strand. The end result of such a reaction is that the single-stranded template nucleic acid is no longer single-stranded; instead, it is base-paired to a synthetic complementary strand. The result is a double-stranded nucleic acid molecule; the original template nucleic acid and its synthetic complementary strand, attached to a hairpin nucleic acid.

The template nucleic acid can then be recovered according to the invention, that is, the complementary strand can be removed by contacting the double-stranded nucleic acid molecule plus hairpin with a nicking endonuclease that is capable of recognizing the restriction site that is in the hairpin nucleic acid, near what was its original 3' end. Because the restriction site is situated so that the nicking endonuclease will create a "nick" at a point near, at, or beyond the original 3' end of the hairpin nucleic acid, the nick will be made before, at, or just beyond, the junction between what was originally the 3' end of the hairpin, and the start of the strand complementary to the template nucleic acid (see, *e*.*g*., Fig. 1).

When a nick is introduced, the sequence distal to the cleavage is no longer contiguous with the sequence proximal to it. That is, the hairpin and the synthetic complementary strand are no longer contiguous. Rather, the synthetic complementary strand effectively becomes a separate, discrete single strand of nucleic acid that is hybridized to the template nucleic acid. The synthetic complementary strand is thus amenable to being washed away by denaturing the overall nucleic acid complex by using heat or chaotropic conditions such as high concentrations of salt. After the synthetic strand is washed away, the template nucleic acid is still attached to the hairpin, and is available for re-sequencing.

Although one embodiment described above uses a hairpin containing a single restriction site for a nicking endonuclease, the sequence of the hairpin can be designed to contain multiple restriction sites, *e*.*g*., for nicking endonucleases or other types of enzymes, such as blunt end endonucleases and/or ordinary restriction enzymes.

For instance, the hairpin can contain restriction sites for both a nicking endonuclease and a blunt end endonuclease. With such a hairpin, one can choose to either recover the template by selectively removing the synthetic complement, as described above, or by use of the blunt end endonuclease, to remove both the synthetic complement and the template, leaving only the hairpin.

The use of a 'nicking' class of enzyme to regenerate the template DNA on an arrayed surface, or a Type IIs endonuclease to regenerate a blunt hairpin, is described. Both of these enzymes may share a common restriction site, or may use different restriction sites. Two of the enzymes discussed herein, N.*Bst*NBI and *Mly*I, exemplify two enzymes that share a common restriction site. In this case, the two enzymes recognize the same sequence of nucleotides, but actually leave at different locations. In the case of enzymes that do not share a common restriction site, the different restriction sites can be included in the design of the hairpin/anchor sequence.

The hairpin nucleic acids or double-stranded nucleic acid anchors can be used to recover the original template in an array, *e*.*g*., a device where multiple nucleic acid sequences are attached to a substrate, *e*.*g*., a device in which fragments of nucleic acid, *e*.*g*., DNA, from a genome of interest are attached to the surface of a glass slide by ligation to a DNA hairpin.

An advantage of the ability to regenerate a template is that a second and subsequent round of sequencing on the same template should eliminate any random sequencing errors that arose during the first round of sequencing. The method is therefore useful in confirming sequencing data.

In general, the hairpins and anchors are useful in situations where a single-stranded nucleic acid template has been made double-stranded, *e*.*g*., in a sequencing reaction, and there is then a need to remove the complementary strand that was synthesized and attached to the template.

Such a sequencing method is illustrated in Fig. 2. The sequence of bases in a template strand is determined by employing a polymerase enzyme to synthesize a complementary strand on the template strand one base at a time. Fig. 2 shows a substrate with a hairpin attached, and a template strand (with the nucleotides represented by circles and squares) attached to one of the ends of the hairpin. Individual bases are then added, each labeled with a different label, *e*.*g*., each with a different fluorophore. One complementary base is attached to the end of the hairpin (or end of the growing synthetic strand) by incorporation, *e*.*g*., by a polymerase, to the growing complementary strand. The identity of the complementary nucleotide is then determined by detection of the fluorophore, *e*.*g*., by washing away unincorporated labeled nucleotides and subsequent detection of the attached fluorophore. The label is then cleaved off the recently-incorporated nucleotide, *e*.*g*., by chemical means, and a nucleotide complementary to the next nucleotide in the template is incorporated into the growing complementary strand, the label detected and identified, and then cleaved off. Subsequent cycles of incorporation, detection and cleavage result in the sequencing of the complementary strand, and perforce, the deduction of the sequence of the original template nucleic acid. Fig. 2 shows the template attached to a hairpin, but the template could alternatively be attached to a segment of double-stranded nucleic acid, *e*.*g*., a double-stranded nucleic acid anchor.

After a series of such incorporations, the original template strand is no longer single stranded, instead, it is base-paired to a growing synthetic complementary strand. Eventually, the template strand may become entirely double-stranded. The hairpins and anchors enable both reuse of the device by recovery and further interrogation of the sequenced template nucleic acid by removal of the synthetic complementary strand, or regeneration of the blunt hairpins on the solid substrate.

The hairpin nucleic acid used to attach the single-stranded template to the solid substrate has been designed such that it contains within its sequence a restriction site for a nicking endonuclease. A "nicking endonuclease" is one of a class of enzymes that bind reversibly to a specific site in double-stranded nucleic acid and then cleave a phosphodiester bond in only one strand at a short distance from the enzyme's binding site. The result is a 'nick' in one strand of the double-stranded nucleic acid, rather than cleavage of both strands. In general, the nicks occur at the 3'-hydroxyl, 5'-phosphate. When a nick is produced in a section of double-stranded nucleic acid, the sequence distal to the restriction site and cleavage site is no longer contiguous with the main body of the double-stranded nucleic acid. It becomes, in essence, a single strand hybridized to the rest of the nucleic acid. It can therefore be washed away by denaturing the nucleic acid using heat or by using chaotropic conditions such as high concentrations of urea.

Several enzymes are known to nick DNA in a single strand but most are found in multiple protein complexes involved in DNA replication or in DNA repair, and as such, have before now had limited applications in manipulating DNA *in vitro.* However, a number of these enzymes are commercially available and can be used to nick DNA under simple reaction conditions. For example, N.*Bst*NBI (available from New England Biolabs, Beverly, Massachusetts, USA) has been used to prepare substrates for studies into DNA repair mechanisms. This and other such enzymes are shown in Table 1, below. A number are available commercially (*e*.*g*., N.AlwI, N.BstNBI, N.BbvCIA and N.BbvCIB are available from New England BioLabs, Inc., Beverly, Massachusetts, USA). Information on enzymes and their cleavage sites can be found in the relevant scientific literature, and/or in public databases, *e*.*g*., REBASE (Robert et al., 2001, Nucl. Acids Res. 29:268-269) ("rebase/"), which is maintained by New England Biolabs on its web site ("neb.com").

**Table 1. Nicking endonucleases and their restriction sites.**

| Enzyme | Restriction Site (5' to 3') | Isoschizomers |
|---|---|---|
| N.AlwI | GGATCNNNN^ | |
| N.BbvCIA | GC^TGAGG | |
| N.BbvCIB | CC^TCAGC | |
| N.Bpu10IA | GC^TNAGG | |
| N.Bpu10IB | CC^TNAGC | |
| N.BspD6I | GAGTCNNNN^ | N.Bst9I N.BstNBI N.BstSEI N.MlyI |
| N.Bst9I | GAGTCNNNN^ | N.BspD6I N.BstNBI N.BstSEI N.MlyI |
| N.BstNBI | GAGTCNNNN^ | N.BspD6I N.Bst9I N.BstSEI N.MlyI |
| N.BstSEI | GAGTCNNNN^ | N.BspD6I N.Bst9I N.BstNBI N.MlyI |
| N.CviPII | C^CD | |
| N.CviQXI | R^AG | |
| N.MlyI | GAGTCNNNNN^ | |

The position of the restriction site of the nicking endonuclease can be chosen so that the enzyme cleaves the synthetic complementary strand from the main body of the hairpin and genomic template stand. After this detached section is washed away, the template strand remains attached to the hairpin and is available for re-sequencing or other applications.

N.*Bst*NBI recognizes the asymmetric sequence GAGTC (SEQ ID NO:1) in double stranded DNA and nicks between the fourth and fifth base downstream of this sequence in the same strand. As described herein, this restriction site has been incorporated into the 3' end of DNA hairpins such that the N.*Bst*NBI enzyme nicks the hairpin just upstream of the synthetic complementary strand, thereby detaching it from the hairpin.

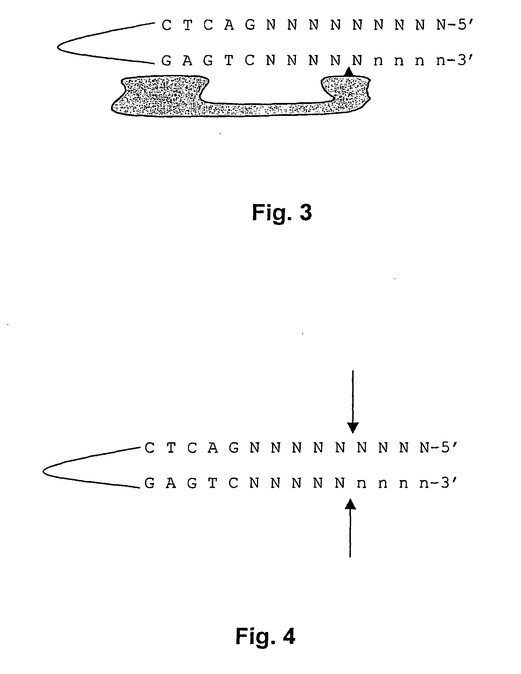

Such a hairpin is shown in Fig. 3. The linear sequence of the hairpin is 5'-NNNNGACTC ... (hairpin loop) ... GAGTCNNNN-3'. The four nucleotides represented by "n" on the lower strand represent the synthesized nucleotides complementary to the four template sequence nucleotides represented by "N" on the upper strand. The enzyme N.*Bst*NBI will nick the complementary strand at the position indicated by the arrow, thereby releasing the lower sequence "nnnn".

The incorporation of this particular restriction site into the hairpin has an added advantage in that it is also recognized by another endonuclease, *Mly*I. In contrast to N.*Bst*NBI, this enzyme cleaves the hairpin in both strands between the fifth and sixth base downstream of the restriction site to produce a blunt end. Thus, the addition of this enzyme following a sequencing reaction on a hairpin allows the original blunt hairpin to be regenerated, as is shown in Fig. 4.

"Blunt end endonucleases" are those which hydrolyze both strands of a nucleic acid, and do so without leaving an overhanging end. A number of blunt end endonucleases are listed in Table 2, below.

**Table 2. Blunt end endonucleases (Type II).**

| Enzyme | Restriction Site (5' to 3') | Isoschizomers |
|---|---|---|
| AhaIII | TTT^AAA | DraI PauAII SruI |
| AluI | AG^CT | MltI |
| BalI | TGG^CCA | MlsI Mlu31I MluNI MscI Msp20I |
| BfrBI | ATG^CAT | |
| BloHII | CTGCA^G | |
| BsaAI | YAC^GTR | BstBAI MspYI PsuAI |
| BsaBI | GATNN^NNATC | Bse8I BseJI Bsh1365I BsiBI BsrBRI MamI |
| BsrBI | CCG^CTC | AccBSI BstD102I Bst31NI MbiI |
| BtrI | CAC^GTC | BmgBI |
| Cac8I | GCN^NGC | BstC8I |
| CviJI | RG^CY | CviTI |
| CviRI | TG^CA | HpyCH4V HpyF44III |
| Eco47III | AGC^GCT | AfeI AitI Aor51HI FunI |
| Eco78I | GGC^GCC | EgeI EheI SfoI |
| EcoICRI | GAG^CTC | Ecl136II Eco53kI MxaI |
| EcoRV | GAT^ATC | CeqI Eco32I HjaI HpyCI NsiCI |
| EsaBC3I | TC^GA | |
| FnuDII | CG^CG | AccII BceBI BepI Bpu95I Bsh1236I Bsp50I Bsp123I BstFNI BstUI Bsu1532I BtkI Csp68KVI CspKVI FalII FauBII MvnI ThaI |
| FspAI | RTGC^GCAY | |
| HaeI | WGG^CCW | |
| HaeIII | GG^CC | BanAI BecAII Bim19II Bme361I BseQI BshI BshFI Bsp211I BspBRI BspKI BspRI BsuRI BteI CltI DsaII EsaBC4I FnuDI MchAII MfoAI NgoPII NspLKI PalI Pde133I PflKI PlaI SbvI SfaI SuaI |
| HindII | GTY^RAC | HinJCI HincII |
| HpaI | GTT^AAC | BstEZ359I BstHPI KspAI SsrI |
| Hpy8I | GTN^NAC | HpyBII |
| LpnI | RGC^GCY | Bme142I |
| MlyI | GAGTCNNNNN^ | SchI |
| MslI | CAYNN^NNRTG | SmiMI |
| MstI | TGC^GCA | Acc16I AosI AviII FdiII FspI NsbI PamI Pun14627I |
| NaeI | GCC^GGC | CcoI PdiI SauBMKI SauHPI SauLPI SauNI SauSI Slu1777I |
| NlaIV | GGN^NCC | AspNI BscBI BspLI PspN4I |
| NruI | TCG^CGA | Bsp68I Mlu2I Sbo13I SpoI |
| NspBII | CMG^CKG | MspA1I |
| OliI | CACNN^NNGTG | AleI |
| PmaCI | CAC^GTG | AcvI BbrPI BcoAI Eco72I PmlI |
| PmeI | GTTT^AAAC | MssI |
| PshAI | GACNN^NNGTC | BoxI BstPAI |
| PsiI | TTA^TAA | |
| PvuII | CAG^CTG | BavI BavAI BavBI Bsp153AI BspM39I BspO4I Cfr6I DmaI EclI NmeRI Pae17kI Pun14627II Pvu84II |
| RsaI | GT^AC | AfaI HpyBI PlaAII |
| ScaI | AGT^ACT | Acc113I AssI DpaI Eco255I RflFII |
| SciI | CTC^GAG | |
| SmaI | CCC^GGG | CfrJ4I PaeBI PspALI |
| SnaBI | TAC^GTA | BstSNI Eco105I |
| SrfI | GCCC^GGGC | |
| SspI | AAT^ATT | |
| SspD5I | GGTGANNNNNNNN^ | |
| StuI | AGG^CCT | AatI AspMI Eco147I GdiI PceI Pme55I SarI Sru30DI SseBI SteI |
| SwaI | ATTT^AAAT | BstRZ246I BstSWI MspSWI SmiI |
| XcaI | GTA^TAC | BspM90I BssNAI Bst1107I BstBSI BstZ17I |
| XmnI | GAANN^NNTTC | Asp700I BbvAI MroXI PdmI |
| ZraI | GAC^GTC | |

It is to be understood that the enzymes used in the invention can be those discovered in nature (*i*.*e*., naturally-occurring enzymes), or can be enzymes created by mutation of existing enzymes.

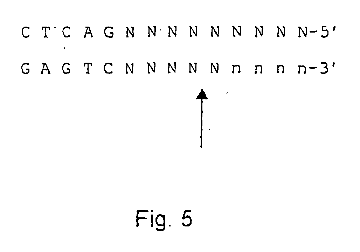

The regeneration protocol is not restricted solely to arrays containing hairpin DNA molecules or DNA molecules constructed on hairpins (*e*.*g*., ligated genomic DNA). Instead, the template can be attached to a double-stranded nucleic acid "anchor" that incorporates the restriction site(s). Such an embodiment is shown in Fig. 5 for the N.*Bst*NBI enzyme.

The hairpins and anchors can be used on double-stranded arrays formed by hybridization of complementary sequences to a single-stranded array, for example, hybridization of a PCR product generated from primers containing a restriction site for a nicking enzyme. Furthermore, the protocol can be applied to other types of arrays besides single-molecule arrays, *i*.*e*., arrays where multiple copies of the same DNA molecule are present at the same locus on the chip.

The hairpin/anchor can also be designed to include one or more restriction sites for nicking endonucleases, blunt end endonucleases, or restriction endonucleases.

For instance, the enzyme N.*Bst*NBI recognizes the sequence 5'-GAGTC-3', and acts by cleaving the strand between four and five nucleotides in the 3' direction from this sequence. This sequence can be incorporated into the hairpin:
5'-NNNNGACTC ... GAGTCNNNN-3',
where "..." represents a number of nucleotides or other moieties added to form the "loop" of the hairpin. Because a hairpin sequence cannot immediately turn upon itself, it is preferable to add 1 to 1000 nucleotides that will form the curve of the loop between the complementary portions of the sequence, preferably 1 to 100 nucleotides.

The *Mly*I restriction site can be "added" to the above sequence by merely adding an extra nucleotide:
5'-NNNNNGACTC ... GAGTCNNNNN-3'.
This sequence would form the hairpin: 
2
┌CTCAGNNNN N▼-5′
└GAGTCNNNN▲N▲-3′
1 2
 where, when the sequence has formed a hairpin, the arrow "1" indicates the site of the nick made by N.*Bst*NBI, and the arrow "2" indicates the site on each "strand" that is cut by *Mly*I.

One can also make use of enzymes that do not recognize the same site. For instance, the blunt end endonuclease SspD5I recognizes the sequence 5'-GGTGANNNNNNNN^-3'. this site can be added into the hairpin shown above by overlapping the end of the SspD5I site with the N.*Bst*NBI and *Mly*I sites: 
2,3
┌CCACTCATNNNN N▼-5′
└GGTGAGTCNNNN▲N▲-3′
1 2,3
 where the arrow "1" indicates the site of the nick made by N.*Bst*NBI, and the arrow "2,3" indicates the site on each "strand" that is cut by either *Mly*I or SspD5I.

There is no requirement that the cleavage sites of one or more of the enzyme be in common, and a number of different sites can be incorporated into the same sequence. For instance, the following sequence 
5'-GAGTC▲NAC▲C▲D▲-3'
      3    4 1 2
 has a nicking site for N.*Bst*NBI (restriction site GAGTCNNNN^) at the arrow "1", a cleavage site for the blunt cutter *Mly*I (restriction site GAGTCTINNNNN^) at arrow "2", a cleavage site for the blunt cutter *Hpy*8I (restriction site GTN^NAC) at arrow "3", and a nicking site at arrow "4" for N.*Cvi*PII (restriction site C^CD). Thus, a variety of restriction sites can be designed into the hairpin or anchor.

The hairpin can also be designed to have an overhang, that is, one "strand" can be longer than the other. This increases the number of possible restriction sites that can be designed into the hairpin. For instance, the hairpin: 
┌CTCAGNACCGGT-5′
└GAGTCNTGG-3′
 can have a nucleic acid template added to its 5' end: 
┌CTCAGNACCGGTNNNN . . . -5′
└GAGTCNTGG -3′.
 Synthesis of the complementary strand will produce the following double-stranded nucleic acid: 
2   3
┌CTCAGNACC G▼GT▼NNNN . . . -5′
└GAGTCNTGG▲C▲CA▲NNNN . . . -3′
1 2   3
 which can be nicked at position 1 by N.*Bst*NBI, and is cleavable across both strands at position 2 by *Mly*I, and at position 3 by *Bol*I, another blunt cutter with restriction site TGG^CCA. The single stranded template can be removed by use of N.*Bst*NBI, or the original hairpin can be recovered by using *Bal*I, followed by N.*Bst*NBI to recover the overhang. Alternatively, a new type of blunt hairpin can be made by incorporating "CCA" onto the 3' end of the hairpin to make it completely double-stranded.

Such overhangs can also be added to blunt hairpins by adding the overhang in the same way one would add a single-stranded nucleic acid template. This can be used to engineer a variety of restriction sites into the new hairpin. The actual template can then be added to the new overhang.

All of the hairpins and methods for designing such hairpins, as discussed above, can also be synthesized in the form of double-stranded nucleic acid "anchors", to be attached to a solid substrate, and to serve as an intermediate molecule anchoring the template to the solid substrate.

All of the sequences described above have had restriction sites designed into the 5' to 3' strand of the hairpin/anchor, with the 5' end of the restriction site being closest to the substrate or anchoring point. Alternatively, however, this can be reversed. If one wished to use an enzyme that operates in the 3' to 5' direction, the sites can be designed into the other "strand" of the hairpin or the other strand of the anchor.

The sites to be designed into the hairpins and anchors can be chosen for a variety of reasons, including an enzyme's specificity or non-specificity, ease of use, longevity, etc.

Alternatively, one can use enzymes that cleave beyond the 5' end of their recognition sites. Enzymes for use in this way can be those discovered in nature (*i*.*e*., naturally-occurring enzymes), or can be created by mutation of existing enzymes. Such enzymes include, *e*.*g*., *Bcg*I, *Bsa*XI and *Bss*KI. *Bss*KI, for example, cleaves as follows:
5'... ^CCNGG ...3'
3'... GGNCC^ ...5'
A mutant of *Bss*KI (or another enzyme) can be made which cleaves in only one strand. This site can be included in a hairpin or anchor as described herein, where the hairpin or anchor has non-cleavable phosphorothioate bonds on the 5' half of the hairpin, so that cleavage only occurs in the 3' half of the hairpin, thereby creating a nick.

In another embodiment, the hairpin nucleic acid or double-stranded nucleic acid anchor can be designed so that the portion to which the template nucleic acid is attached contains non-cleavable bonds. That is, in the portion of the hairpin/anchor to which the template nucleic acid is attached, the nucleotides are attached to each other by bonds which are not cleavable by an endonuclease. In such a hairpin/anchor, an ordinary restriction endonuclease can be used, but it will behave as a nicking endonuclease, and will cleave only one strand -- the one with the cleavable bonds between the nucleotides.

The non-cleavable bonds can be phosphorothioate bonds, which are easily added during the synthesis of the hairpin/anchor. Any modification of the phosphodiester backbone of the hairpin/anchor can be used, where the modification allows binding of the restriction endonuclease to the hairpin/anchor, but prevents cleavage of the strand containing the modifications.

For instance, *Aat*II normally cleaves the following sequence:
5'... G-A-C-G-T^C...3'
3'... C^T-G-C-A-G...5'
However, if the normal bonds ("-") between the nucleotides at one of the cleavage cites were replaced with bonds that are not cleavable ("=") by *Aat*II, then the cleavage pattern would resemble that of a nicking endonuclease:
5'... G-A-C-G-T=C ...3'
3'... C^T-G-C-A-G ...5'

The use of endonucleases facilitates simple cleaving of the DNA at an exact position in natural DNA bases. Therefore, no additional costs are incurred in constructing the hairpin/anchor sequences. Furthermore, the use of an endonuclease guarantees that DNA cleavage produces termini that are substrates for further manipulation by other enzymes such as ligases or polymerases.

Regeneration of single-stranded DNA templates on a sequencing chip or nucleic acid array produces a spatially addressable array where the sequence of DNA at every position on the array is known. Such an array can be treated with a polymerase enzyme and natural dNTPs to produce a double-stranded array that is also spatially addressable enabling the systematic analysis of DNA-protein interactions.

The density of the single molecule arrays is not critical. However, the present invention can make use of a high density of hairpins/anchors, and these are preferable. For example, arrays with a density of 10⁶-10⁹ hairpins/anchors per cm² may be used. Preferably, the density is at least 10⁷/cm² and typically up to 10⁹/cm². These single molecule arrays are in contrast to other arrays which may be described in the art as "high density" but which are not necessarily as high and/or which do not allow single molecule resolution.

Using the methods and devices of the present invention, it may be possible to image at least 10⁶ - 109, preferably 10⁷ or 10⁸ hairpins or anchors per cm². Fast sequential imaging may be achieved using a scanning apparatus; shifting and transfer between images may allow higher numbers of hairpins/anchors to be imaged.

The extent of separation between the individual hairpins/anchors on the array will be determined, in part, by the particular technique used to resolve the individual hairpins/anchors. Apparatus used to image molecular arrays are known to those skilled in the art. For example, a confocal scanning microscope may be used to scan the surface of the array with a laser to image directly a fluorophore incorporated on the individual hairpins/anchors by fluorescence. Alternatively, a sensitive 2-D detector, such as a charge-coupled device, can be used to provide a 2-D image representing the individual hairpins/anchors on the array.

"Resolving" single hairpins/anchors (and their attached templates and complements) on the array with a 2-D detector can be done if, at 100 x magnification, adjacent hairpins/anchors are separated by a distance of approximately at least 250 nm, preferably at least 300 nm and more preferably at least 350 nm. It will be appreciated that these distances are dependent on magnification, and that other values can be determined accordingly, by one of ordinary skill in the art.

Other techniques such as scanning near-field optical microscopy (SNOM) are available which are capable of greater optical resolution, thereby permitting more dense arrays to be used. For example, using SNOM, adjacent hairpins/anchors may be separated by a distance of less than 100 nm, *e*.*g*., 10 nm. For a description of scanning near-field optical microscopy, see Moyer et al., Laser Focus World (1993) 29(10).

An additional technique that may be used is surface-specific total internal reflection fluorescence microscopy (TIRFM); see, for example, Vale et al., Nature (1996) 380:451-453). Using this technique, it is possible to achieve wide-field imaging (up to 100 µm x 100 µm) with single molecule sensitivity. This may allow arrays of greater than 10⁷ resolvable hairpins/anchors per cm² to be used.

Additionally, the techniques of scanning tunnelling microscopy (Binnig et al., Helvetica Physica Acta (1982) 55:726-735) and atomic force microscopy (Hansma et al., Ann. Rev. Biophys. Biomol. Struct. (1994) 23:115-139) are suitable for imaging the arrays of the present invention. Other devices which do not rely on microscopy may also be used, provided that they are capable of imaging within discrete areas on a solid support.

Immobilisation to the support may be by specific covalent or non-covalent interactions. Covalent attachment is preferred. The immobilized hairpin/anchor is then able to undergo interactions with other molecules or cognates at positions distant from the solid support. Immobilisation in this manner results in well separated hairpins/anchors. The advantage of this is that it prevents interaction between neighbouring hairpins/anchors on the array, which may hinder interrogation of the array.

An array containing sequenced and regenerated templates can be used as an addressable platform for spatially organizing libraries of compounds attached to single stranded DNA tags. For example, a combinatorial library of drug compounds could be prepared with unique single stranded DNA tags or DNA mimics, *e*.*g*., PNA, and then added to a sequenced/regenerated array. This would generate a spatially addressable array of drug compounds on a chip. The same can be done for a protein library. Such chips could then be interrogated with probes to generate information about molecular interactions.

The arrays described herein are effectively single analyzable template nucleic acids. This has many important benefits for the study of the template sequences and their interaction with other biological molecules. In particular, fluorescence events occurring on each template nucleic acid can be detected using an optical microscope linked to a sensitive detector, resulting in a distinct signal for each template.

When used in a multi-step analysis of a population of single templates, the phasing problems (loss of synchronisation) that are encountered using high density (multi-molecule) arrays of the prior art, can be reduced or removed. Therefore, the arrays also permit a massively parallel approach to monitoring fluorescent or other events on the templates. Such massively parallel data acquisition makes the arrays extremely useful in a wide range of analysis procedures which involve the screening/characterising of heterogeneous mixtures of templates.

### Example 1: Regeneration of Hairpin.

Twenty microliters of solution is prepared containing 50 pmoles of a DNA hairpin phosphorylated at its 5' end, 10 pmoles of a non-phosphorylated DNA double-stranded oligonucleotide, and several thousand units of a DNA ligase enzyme. The oligonucleotide is designed such that one strand is shorter than the other, making the oligonucleotide blunt-ended at one end and single stranded at the other, a 5' end. The single-stranded end carries a fluorescent label. The action of the ligase enzyme fuses the hairpin and the double-stranded oligonucleotide at their blunt ends only, and because only the 5' end of the hairpin carries a phosphate group, the reaction results in joining one strand to the hairpin - the longer strand that carries the fluorescent group.

The template is regenerated by taking a solution containing 2.5 pmoles of a fluorescently labeled strand of DNA that has been previously ligated to a blunt DNA hairpin. The single-stranded portion of this DNA construct, *i*.*e*., the template strand, can be made double-stranded by employing 1 Unit of Vent exo⁻ polymerase (New England Biolabs, Inc., Beverly, Massachusetts, USA) to incorporate a mixture of four oligonucleotides, each at a concentration of 25 pmoles per reaction, at 75°C for 30 minutes. Upon completion, the reaction mixture is purified using a DNA purification kit (Qiagen, Hilden, Germany) and split in two. Half is kept for analysis and half (1.25 pmoles) is digested at 55°C for 30 minutes with N.*Bst*NBI (5 Units; New England Biolabs, Inc., Beverly, Massachusetts, USA), which nicks the extended DNA construct proximal to the new synthetic stand. The formation of the synthetic complementary strand by the polymerase enzyme and its removal by digestion with the nicking enzyme can be analyzed by polyacrylamide gel electrophoresis, which distinguishes the DNA products by virtue of their differences in size. The presence of the fluorescent group ensures that the DNA molecules can be easily detected.

This procedure can also be performed with little modification in a flow-cell where the substrate comprises DNA ligated to DNA hairpins that are covalently attached to the glass surface of the flow cell. In this case, the attachment of the DNA to a solid support, the glass, obviates the need to employ a DNA purification kit between enzyme steps: instead, products can be removed and new reagents added by flowing solutions across through the cell.

### Example 2. Bisulfite Reaction.

In general, the DNA is rendered single-stranded by taking a 20 µl solution of 2-10 µg of genomic DNA fragments and adding 0.3M NaOH and incubating at room temperature for 15 minutes. 150 µl of 0.6 M hydroquinone containing 3.5 M sodium bisulfite (pH 5) is then added, and the mixture incubated for 10 hours at 50°C. The reaction is then purified using a DNA purification kit (Qiagen, Hilden, Germany).

When performing the bisulfite reaction on DNA on an array, prior denaturation of the DNA is not required. The DNA will be single stranded and attached to a hairpin nucleic acid or a double-stranded nucleic acid anchor on a surface. The DNA will have been rendered single-stranded after a sequencing reaction by the action of a nicking endonuclease that cleaves the sequencing strand away from the immobilised template strand. Thus, a 150 µl solution of 0.6 M hydroquinone containing 3.5 M sodium bisulfite (pH 5) is injected onto the array, and the array is then incubated at 50°C for 5 hours. The array is then washed with water, then 150 µl of 200 mM NaOH added and incubated for 20 minutes. The array is next washed with 1 ml of 200 mM HCl, then finally washed with 5 ml of water. The array is then ready for a second round of sequencing to determine the methylation status of the DNA on the array.

While this invention has been particularly shown and described with references to preferred embodiments thereof, it will be understood by those skilled in the art that various changes in form and details may be made therein without departing from the scope of the invention encompassed by the appended claims.

## Claims

1. A method for detecting a methylated cytosine in a template nucleic acid, the method comprising:
(a) providing a hairpin-template complex, comprising:
(i) a hairpin nucleic acid, wherein the hairpin nucleic acid is self-complementary and has a first restriction site for a nicking endonuclease, said restriction site comprising a recognition sequence and a cleavage site, wherein said recognition sequence is situated so that said cleavage site is before, at, or beyond the 3' end of the hairpin nucleic acid, and wherein said hairpin nucleic acid is a self-hybrid; and
(ii) a single-stranded template nucleic acid;
wherein the 5' end of the hairpin nucleic acid is attached to the 3' end of the single-stranded template nucleic acid;
(b) sequencing the single-stranded template nucleic acid of the hairpin-template complex, thereby producing:
(i) (ii) a first sequence; and
(ii) (i) a hairpin-template-complement complex, comprising the hairpin-template complex of (a), and further comprising a synthetic nucleic acid strand complementary to the template nucleic acid, wherein the synthetic nucleic acid strand is hybridized to the template nucleic acid, and wherein the complementary nucleic acid strand is attached at its 5' end to the 3' end of the hairpin nucleic acid;
(c) removing the complementary nucleic acid strand from the hairpin-template-complement complex, thereby recovering the hairpin-template complex, wherein the removal is carried out by a process comprising the use of the said nicking restriction endonuclease which binds to said recognition site in said hairpin and cleaves the hairpin-template-complement complex at a site between the hairpin and the template complement;
(d) treating the hairpin-template complex with sodium bisulfite, thereby producing a sodium bisulfite-treated template nucleic acid;
(e) sequencing the sodium bisulfite-treated template nucleic acid of (c), thereby producing a second sequence; and
(f) comparing the first sequence and the second sequence, where the presence of a cytosine in the second sequence indicates that the cytosine at that position is methylated;
thereby detecting a methylated cytosine in the template nucleic acid.

2. The method of claim 1, wherein the hairpin nucleic acid is attached to a solid support.

3. The method of claim 2 wherein the hairpin nucleic acid forms part of an array comprising a plurality of hairpin nucleic acids distributed over the solid support.

4. The method of claim 3 wherein adjacent hairpin nucleic acids on the array are separated by a distance of at least 10nm.

5. The method of claim 3, wherein the hairpin nucleic acids are separated by a distance of at least 100nm.

6. The method of claim 3, wherein the hairpin nucleic acids are separated by a distance of at least 250nm.

7. The method of any of claims 3 to 6, wherein the density of the hairpin nucleic acids is from 10⁶ to 10⁹ polynucleotides per cm².

8. The method of any of claims 3 to 6, wherein the density of the hairpin nucleic acids is from 10⁷ to 10⁸ molecules per cm².

9. A method for detecting a methylated cytosine in a template nucleic acid, the method comprising:
(a) providing an anchor-template complex, comprising:
(i) a double-stranded nucleic acid anchor, wherein the double-stranded nucleic acid anchor comprises:
(A) a first end and a second end; and
(B) a first restriction site for a nicking endonuclease, said restriction site comprising a recognition sequence and a cleavage site, wherein said cleavage site is situated so that said cleavage site is before, at, or beyond the 3' end of the first end of the double-stranded nucleic acid anchor; and
(ii) a single-stranded template nucleic acid;
wherein the 5' end of the first end of the double-stranded nucleic acid anchor is attached to the 3' end of the single-stranded template nucleic acid;
(b) sequencing the single-stranded template nucleic acid of the anchor-template complex, thereby producing:
(i) a first sequence; and
(ii) an anchor-template-complement complex, comprising the anchor-template complex of (a), and further comprising a synthetic nucleic acid strand complementary to the template nucleic acid, wherein the synthetic nucleic acid strand is hybridized to the template nucleic acid, and wherein the complementary nucleic acid strand is attached at its 5' end to the 3' end of the first end of the double-stranded nucleic acid anchor;
(c) removing the complementary nucleic acid strand from the anchor-template-complement complex, thereby recovering the anchor-template complex, wherein the removal is carried out by a process comprising the use of the said nicking restriction endonuclease which binds to said recognition site in said anchor and cleaves the anchor-template-complement complex at a site between the anchor and the template complement;
(d) treating the anchor-template complex with sodium bisulfite, thereby producing a sodium bisulfite-treated anchor-template complex;
(e) sequencing the sodium bisulfite-treated anchor-template complex of (d), thereby producing a second sequence; and
(f) comparing the first sequence and the second sequence, where the presence of a cytosine in the second sequence indicates that the cytosine at that position in the template nucleic acid is methylated;
thereby detecting a methylated cytosine in the template nucleic acid.

10. The method of claim 9, wherein the double-stranded nucleic acid anchor is attached to a solid support.

11. The method of claim 10 wherein the double-stranded nucleic acid anchor forms part of an array comprising a plurality of double-stranded anchors distributed over the solid support.

12. The method of claim 11 wherein the array comprises a plurality of individual addresses, wherein each individual address on the array comprises a cluster of nucleic acids of the same type.

## Patentansprüche

1. Verfahren zum Nachweis eines methylierten Cytosins in einer Template-Nukleinsäure, wobei das Verfahren Folgendes umfasst:
(a) Bereitstellen eines Haarnadeltemplate-Komplexes, der Folgendes umfasst:
(i) eine Haarnadel-Nukleinsäure, wobei die Haarnadel-Nukleinsäure selbstkomplementär ist und eine erste Restriktionsstelle für eine Nicking-Endonuklease aufweist, wobei die Restriktionsstelle eine Erkennungssequenz und eine Spaltungsstelle umfasst, in der die Erkennungssequenz so angeordnet ist, dass die Spaltungsstelle vor, an oder jenseits des 3'-Endes der Haarnadel-Nukleinsäure liegt, und wobei die Haarnadel-Nukleinsäure ein Selbsthybrid ist; und
(ii) eine einzelsträngige Template-Nukleinsäure;
wobei das 5'-Ende der Haarnadel-Nukleinsäure an das 3'-Ende der einzelsträngigen Template-Nukleinsäure gebunden ist;
(b) Sequenzieren der einzelsträngigen Template-Nukleinsäure des Haarnadeltemplate-Komplexes, wodurch Folgendes erzeugt wird:
(i) eine erste Sequenz; und
(ii) ein Haarnadeltemplate-Komplementkomplex, der den Haarnadeltemplate-Komplex aus (a) und weiterhin einen synthetischen Nukleinsäurestrang umfasst, der komplementär zu der Template-Nukleinsäure ist, wobei der synthetische Nukleinsäurenstrang an die Template-Nukleinsäure hybridisiert ist, und wobei der komplementäre Nukleinsäurestrang an seinem 5'-Ende an das 3'-Ende der Haarnadel-Nukleinsäure gebunden ist;
(c) Entfernen des komplementären Nukleinsäurestrangs von dem Haarnadeltemplate-Komplementkomplex, wodurch der Haarnadeltemplate-Komplex zurückgewonnen wird, wobei das Entfernen anhand eines Verfahrens durchgeführt wird, das die Verwendung der zuvor erwähnten Nicking-Restriktionsendonuklease umfasst, die sich mit der Erkennungsstelle in der Haarnadel verbindet und den Haarnadeltemplate-Komplementkomplex an einer Stelle zwischen der Haarnadel und dem Template-Komplement spaltet;
(d) Behandeln des Haarnadeltemplate-Komplex mit Natriumbisulfit, wodurch eine mit Natriumbisulfit behandelte Template-Nukleinsäure erzeugt wird;
(e) Sequenzieren der mit Natriumbisulfit behandelten Template-Nukleinsäure aus (c), wodurch eine zweite Sequenz erzeugt wird; und
(f) Vergleichen der ersten Sequenz und der zweiten Sequenz, wobei die Anwesenheit eines Cytosins in der zweiten Sequenz anzeigt, dass das Cytosin an dieser Position methyliert ist;
wodurch ein methyliertes Cytosin in der Template-Nukleinsäure nachgewiesen wird.

2. Verfahren nach Anspruch 1, wobei die Haarnadel-Nukleinsäure an einen festen Träger gebunden ist.

3. Verfahren nach Anspruch 2, wobei die Haarnadel-Nukleinsäure einen Teil eines Arrays bildet, der mehrere Haarnadel-Nukleinsäuren umfasst, die auf dem festen Träger verteilt sind.

4. Verfahren nach Anspruch 3, wobei benachbarte Haarnadel-Nukleinsäuren auf dem Array durch eine Entfernung von mindestens 10 nm getrennt sind.

5. Verfahren nach Anspruch 3, wobei die Haarnadel-Nukleinsäuren durch eine Entfernung von mindestens 100 nm getrennt sind.

6. Verfahren nach Anspruch 3, wobei die Haarnadel-Nukleinsäuren durch eine Entfernung von mindestens 250 nm getrennt sind.

7. Verfahren nach einem der Ansprüche 3 bis 6, wobei die Dichte der Haarnadel-Nukleinsäuren zwischen 10⁶ und 10⁹ Polynukleotide pro cm² beträgt.

8. Verfahren nach einem der Ansprüche 3 bis 6, wobei die Dichte der Haarnadel-Nukleinsäuren zwischen 10⁷ und 10⁸ Moleküle pro cm² beträgt.

9. Verfahren zum Nachweis eines methylierten Cytosins in einer Template-Nukleinsäure, wobei das Verfahren Folgendes umfasst:
(a) Bereitstellen eines Ankertemplate-Komplexes, der Folgendes umfasst:
(i) einen doppelsträngigen Nukleinsäureanker, wobei der doppelsträngige Nukleinsäureanker Folgendes umfasst:
(A) ein erstes Ende und ein zweites Ende; und
(B) eine erste Restriktionsstelle für eine Nicking-Endonuklease, wobei die Restriktionsstelle eine Erkennungssequenz und eine Spaltungsstelle umfasst,
wobei die Spaltungsstelle so angeordnet ist, dass die Spaltungsstelle vor, an oder jenseits des 3'-Endes des ersten Endes des doppelsträngigen Nukleinsäureankers liegt und
(ii) eine einzelsträngige Template-Nukleinsäure;
wobei das 5'-Ende des ersten Endes des doppelsträngigen Nukleinsäureankers an das 3'-Ende der einzelsträngigen Template-Nukleinsäure gebunden ist;
(b) Sequenzieren der einzelsträngigen Template-Nukleinsäure des Ankertemplate-Komplexes, wodurch Folgendes erzeugt wird:
(i) eine erste Sequenz; und
(ii) ein Ankertemplate-Komplementkomplex, der den Ankertemplate-Komplex aus (a) und weiterhin einen synthetischen Nukleinsäurestrang umfasst, der komplementär zu der Template-Nukleinsäure ist, wobei der synthetische Nukleinsäurenstrang an die Template-Nukleinsäure hybridisiert ist, und wobei der komplementäre Nukleinsäurestrang an seinem 5'-Ende an das 3'-Ende des ersten Endes des doppelsträngigen Nukleinsäureankers gebunden ist;
(c) Entfernen des komplementären Nukleinsäurestrangs von dem Ankertemplate-Komplementkomplex, wodurch der Ankertemplate-Komplex zurückgewonnen wird, wobei das Entfernen anhand eines Verfahrens durchgeführt wird, das die Verwendung der zuvor erwähnten Nicking-Restriktionsendonuklease umfasst, die sich mit der Erkennungsstelle im Anker verbindet und den Ankertemplate-Komplementkomplex an einer Stelle zwischen dem Anker und dem Template-Komplement spaltet;
(d) Behandeln des Ankertemplate-Komplexes mit Natriumbisulfit, wodurch ein mit Natriumbisulfit behandelter Ankertemplate-Komplex erzeugt wird;
(e) Sequenzieren des mit Natriumbisulfit behandelten Ankertemplate-Komplexes aus (d), wodurch eine zweite Sequenz erzeugt wird; und
(f) Vergleichen der ersten Sequenz und der zweiten Sequenz, wobei die Anwesenheit eines Cytosins in der zweiten Sequenz anzeigt, dass das Cytosin an dieser Position in der Template-Nukleinsäure methyliert ist;
wodurch ein methyliertes Cytosin in der Template-Nukleinsäure nachgewiesen wird.

10. Verfahren nach Anspruch 9, wobei der doppelsträngige Nukleinsäureanker an einen festen Träger gebunden ist.

11. Verfahren nach Anspruch 10, wobei der doppelsträngige Nukleinsäureanker einen Teil eines Arrays bildet, der mehrere doppelsträngige Nukleinsäureanker umfasst, die auf dem festen Träger verteilt sind.

12. Verfahren nach Anspruch 11, wobei der Array mehrere individuelle Adressen umfasst, wobei jede individuelle Adresse auf dem Array ein Cluster von Nukleinsäuren des gleichen Typs umfasst.

## Revendications

1. Une méthode de détection d'une cytosine méthylée dans un acide nucléique matrice, la méthode comprenant:
(a) l'acquisition d'un complexe épingle à cheveux-matrice, comportant:
(i) un acide nucléique matrice en épingle à cheveux, dans lequel l'acide nucléique en épingle à cheveux est auto complémentaire et possède un premier site de restriction pour une endonucléase de coupure, ledit site de restriction comprenant une séquence de reconnaissance et un site de clivage, la séquence dudit site de reconnaissance étant située en amont, à l'endroit même, ou en aval de l'extrémité 3' de l'acide nucléique en épingle à cheveux, et ledit acide nucléique en épingle à cheveux étant un auto-hybride; et
(ii) un acide nucléique matrice àsimple brin;
dans lequel l'extrémité 5' de l'acide nucléique en épingle à cheveux est attachée à l'extrémité 3' de l'acide nucléique matrice àsimple brin;
(b) le séquençage de l'acide nucléique matrice àsimple brin du complexe épingle à cheveux-matrice, produisant ainsi:
(i) une première séquence; et
(ii) un complexe épingle à cheveux-matrice-complément, comprenant le complexe épingle à cheveux-matrice de (a), et comprenant également un brin d'acide nucléique synthétique complémentaire de l'acide nucléique matrice,
dans lequel le brin d'acide nucléique synthétique est hybridé à l'acide nucléique matrice, et dans lequel le brin complémentaire d'acide nucléique est attaché à son extrémité 5' à l'extrémité 3' de l'acide nucléique en épingle à cheveux;
(c) le retrait du brin complémentaire d'acide nucléique du complexe épingle à cheveux-matrice-complément, ce qui engendre la récupération du complexe épingle à cheveux-matrice, dans lequel le retrait est réalisé par un procédé comprenant l'utilisation de ladite endonucléase de restriction de coupure qui lie ledit site de reconnaissance dans ladite épingle à cheveux et clive le complexe épingle à cheveux-matrice-complément en un site placé entre l'épingle à cheveux et le complément de matrice;
(d) le traitement du complexe épingle à cheveux-matrice au bisulfite de sodium, entraînant la production d'un acide nucléique matrice traité au bisulfite de sodium;
(e) le séquençage de l'acide nucléique matrice traité au bisulfite de sodium de (c), entraînant la production d'une deuxième séquence; et
(f) la comparaison de la première séquence et de la deuxième séquence, dans laquelle la présence d'une cytosine dans la deuxième séquence indique que la cytosine à cette position est méthylée;
d'où la détection d'une cytosine méthylée dans l'acide nucléique matrice.

2. La méthode de la revendication 1, dans laquelle l'acide nucléique en épingle à cheveux est attaché à un support solide.

3. La méthode de la revendication 2 dans laquelle l'acide nucléique en épingle à cheveux forme une partie d'une gamme comprenant une pluralité d'acides nucléiques en épingle à cheveux répartis sur le support solide.

4. La méthode de la revendication 3 dans laquelle les acides nucléiques en épingle à cheveux adjacents sur la gamme sont séparés par une distance d'au moins 10 nm.

5. La méthode de la revendication 3, dans laquelle les acides nucléiques en épingle à cheveux sont séparés par une distance d'au moins 100 nm.

6. La méthode de la revendication 3, dans laquelle les acides nucléiques en épingle à cheveux sont séparés par une distance d'au moins 250 nm.

7. La méthode d'une des revendications 3 à 6, dans laquelle la densité des acides nucléiques en épingle à cheveux se situe entre 10⁶ et 10⁹ polynucléotides par cm².

8. La méthode d'une des revendications 3 à 6, dans laquelle la densité des acides nucléiques en épingle à cheveux se situe entre 10⁷ et 10⁸ molécules par cm²

9. Une méthode de détection d'une cytosine méthylée dans un acide nucléique matrice, ladite méthode comprenant:
(a) l'acquisition d'un complexe ancrage-matrice, comportant:
(i) un site d'ancrage d'un acide nucléique à double brins, dans laquelle le site d'ancrage d'acide nucléique à doubles brins comprend:
(A) une première extrémité et une deuxième extrémité; et
(B) un premier site de restriction pour une endonucléase de coupure, ledit site de restriction comprenant une séquence de reconnaissance et un site de clivage, ledit site de clivage étant situé en amont, à l'endroit même ou en aval de l'extrémité 3' de la première extrémité du site d'ancrage de l'acide nucléique à doubles brins; et
(ii) un acide nucléique matrice àsimple brin;
dans lequel l'extrémité 5' de la première extrémité du site d'ancrage d'acide nucléique à doubles brins est attaché à l'extrémité 3' de l'acide nucléique matrice àsimple brin;
(b) le séquençage de l'acide nucléique matrice àsimple brin du complexe ancrage-matrice, produisant ainsi:
(i) une première séquence; et
(ii) un complexe ancrage-matrice, comprenant le complexe ancrage-matrice de (a), et comprenant également un brin d'acide nucléique synthétique complémentaire de l'acide nucléique matrice, dans lequel le brin d'acide nucléique synthétique est hybridé à l'acide nucléique matrice, et dans lequel le brin complémentaire d'acide nucléique est attaché à son extrémité 5' à l'extrémité 3' de la première extrémité du site d'ancrage de l'acide nucléique à doubles brins;
(c) le retrait du brin complémentaire d'acide nucléique du complexe ancrage-matrice-complément, ce qui engendre la récupération du complexe ancrage-matrice, dans lequel le retrait est réalisé par un procédé comprenant l'utilisation de ladite endonucléase de restriction de coupure qui lie ledit site de reconnaissance dans ledit site d'ancrage et clive le complexe ancrage-matrice-complément en un site placé entre le site d'ancrage et le complément de matrice;
(d) le traitement du complexe ancre-matrice au bisulfite de sodium, entraînant la production d'un complexe ancre traitée au bisulfite de sodium-matrice;
(e) le séquençage du site d'ancrage traité au bisulfite de sodium-matrice de (d), entraînant la production d'une deuxième séquence; et
(f) la comparaison de la première séquence et de la deuxième séquence, dans laquelle la présence d'une cytosine dans la deuxième séquence indique que la cytosine en cette position dans l'acide nucléique matrice est méthylée; d'où la détection d'une cytosine méthylée dans l'acide nucléique matrice.

10. La méthode de la revendication 9, dans laquelle le site d'ancrage de l'acide nucléique à doubles brins est attachée à un support solide.

11. La méthode de la revendication 10 dans laquelle le site d'ancrage de l"acide nucléique à doubles brins forme une partie d'une gamme comprenant une pluralité de sites d'ancrage à doubles brins répartis sur le support solide.

12. La méthode de la revendication 11 dans laquelle la gamme comprend une pluralité d'adresses individuelles, dans laquelle chaque adresse individuelle de la gamme comprend un ensemble d'acides nucléiques du même type.
